# EUROPEAN PATENT APPLICATION

(11) **EP 2 092 912 A1**
(43) Date of publication of application: **26.08.2009**
(21) Application number: 08101738.6
(22) Date of filing: 19.02.2008
(51) Int. Cl.: A61F 2/00

(54) **Artificial contractile structure**

(71) Applicant: Nanopowers S.A., 1012 Lausanne (CH)
(72) Inventor: Tozzi, Piergiorgio, 1012 Lausanne (CH); Hayoz, Daniel, 1752 Villars-sur-Glâne (CH); Von Segesser, Ludwig, 1005 Lausanne (CH)
(74) Representative: GLN

(57) **Abstract**

The present invention relates to artificial contractile structures generally devised to be used in the medical field. Such structures may be advantageously used to assist the functioning of an organ, e.g. a sphincter.

The structure (1) comprises a support (2) and several fibers (5) fixed to said support (2), said fibers (5) being made of a contractile material which can be activated by an activator in such a way as to provide said structure in a rest or in an activated position, the rest position being defined with non-rectilinear fibers and the activated position being defined with fibers of reduced length, said structure being adapted to be placed around a tubular part of an organ to be contracted, wherein the fibers (5) are distributed along said support (2) in order to be able to reduce the diameter of said tubular part, when said fibers (5) are contracted, in at least two distinct regions of the tubular part, and wherein the fibers (5) are designed to be activated pulsatory and alternately, so as to completely close at least one region of the tubular part, in a pulsating and alternating manner.

## Description

### Technical Field

The present invention relates to artificial contractile structures generally devised to be used in the medical field. Such structures may be advantageously used to assist the functioning of an organ, e.g. a sphincter. More generally, they can be used for moving a tubular part of the body.

The present application incorporate by reference the complete teaching of international patent application PCT/IB2006/055044 filed by the same applicant.

### Background of the invention

The faecal and urinary incontinences affect 10% of people over 60 years of age and dramatically increase over 80. Several pharmaceutical or surgical solutions have been developed for treating incontinence. Generally, the outcome of surgery for treatment of urinary and faecal incontinence has to be regarded as low. The impacts on health care costs and overall quality of life of the patient are enormous.

Some publications describe the use of artificial sphincters comprising shape memory alloy elements suitable for opening and closing a part of an organ in a living body. EP 1 238 638 describes an artificial sphincter having an opening/closing portion for opening and closing, wherein said opening/closing portion has:
- a pair of elongated shape memory alloy elements that change reversibly between opposite shapes upon changes in temperature, and
- hinges that link said pair of shape memory alloy element together in a cylindrical shape.

Such artificial sphincter is placed around the intestine so that the opening/closing portion constricts the intestine. When the shape memory alloy elements are heated, they change shape, so that the constricting force on the intestine is lost.

However, as the opening/closing portion is still constricting the same region of the intestine, there is a damage to this part of the body, and more especially a risk of burns of the tissue due to the heating of the shape memory alloy elements.

Moreover, in normal state, the shape memory alloy elements are not heated and are each bent to constrict the intestine. That means that heating is necessary to open the artificial sphincter. If the heating means fail, the sphincter remains closed and cannot be open. A surgery is then necessary to open the artificial sphincter and solve the problem.

Another artificial sphincter has been proposed in JP 07-051304. This document describes a constrictor comprising two shape memory alloy elements with different shape memories, and covered by covering materials. The first covering material is formed in a shape to close the urethra in the daytime, and the second covering material is formed in a shape to half closed the urethra in the night. This sphincter allows to change the pressure to the urethra, in order to prevent the incontinence in life action in the daytime, and to avoid necrosis of the tissue by loosing the pressure to the urethra in the night.

However, the drawbacks of such artificial sphincter are that there is a risk of necrosis due to the pressure to the urethra during the day and that there is a risk of incontinence during the night.

Therefore there are, at the present time, no adequate solutions for the treatment of incontinence.

### Summary of the invention

The present invention provides an artificial contractile structure which allows to avoid the disadvantages of the prior art.

In one aspect, the present invention provides an artificial contractile structure which allows to significantly improve sphincter muscle function and to improve also the patient's quality of life with a significant reduction of treatment costs.

Accordingly, an artificial contractile structure according to one aspect of the invention, comprises a support and several fibers fixed to said support, said fibers being made of a contractile material which can be activated by an activator, e.g. an electric current/voltage, in such a way as to provide said structure in a rest or in an activated position, the rest position being defined with non-rectilinear fibers and the activated position being defined with fibers of reduced length, said structure being adapted to be placed around a tubular part of an organ to be contracted, wherein the fibers are distributed along said support in order to be able to reduce the diameter of said tubular part, when said fibers are contracted, in at least two distinct regions of the tubular part, and wherein the fibers are designed to be activated pulsatory and alternately, so as to completely close at least one region of the tubular part, in a pulsating and alternating manner.

Such artificial structure is used for assisting or replacing a natural sphincter, especially for the treatment of faecal or urinary incontinence.

In some embodiments, the fibers have at least two distinct parts fixed to the support.

In other embodiments, the transition from the rest towards the activated position or vice-versa is defined by a fiber movement along a lateral direction which is perpendicular with respect to the fiber length.

In a preferred embodiment, the support is a hinged clamp adapted to be placed around the tubular part, comprising at least two pairs of teeth, wherein a fiber is fixed to the inside of each pair of teeth in order to cross it so as to form at least two gates, each gate being able to constrict one region of the tubular part to close it, in a pulsating and alternating manner.

In another embodiment, the support comprises parallel rods to which at least two fibers are fixed by their ends, so that each fiber forms a loop adapted to be placed around the tubular part, the loops forming at least two gates, each gate being able to constrict one region of the tubular part to close it, in a pulsating and alternating manner.

In another embodiment, the support comprises a U rod to which at least two fibers are fixed by their central part, so that each fiber forms a U adapted to be placed around the tubular part, the U fibers forming at least two gates, each gate being able to constrict one region of the tubular part to close it, in a pulsating and alternating manner.

In another embodiment, the support comprises a housing adapted to be placed around the tubular part, said housing being closed by a sliding element, and wherein at least two fibers are suspended by their ends to the support, the central part of each fiber comprising a press pad placed opposite the sliding element, the fibers and their press pads forming at least two gates, each gate being able to constrict one region of the tubular part to close it, in a pulsating and alternating manner.

In another aspect, the present invention provides an apparatus comprising an artificial contractile structure as described above, and further comprising an activator adapted to pulsatory and alternately contract the fibers so that said fibers completely close at least one region of the tubular part, in a pulsating and alternating manner, and means for opening on demand said artificial contractile structure.

In some embodiments, the apparatus further comprises sensing means selected from pressure, temperature and movement sensing means. Said sensing means can be fixed to the support of the artificial contractile structure or may even form part of it.

In other embodiments, the apparatus further comprises an implantable source of energy.

### Brief description of the drawings

Figure 1 is a side view of a first embodiment of the artificial structure according to the present invention,

Figure 2 is a cross-sectional view of structure of Figure 1, in open position,

Figure 3 is a cross-sectional view of structure of Figure 1, in closed position around a tubular organ, the fibers being inactivated,

Figure 4 is a cross-sectional view of structure of Figure 1, in closed position around a tubular organ, the fibers being activated,

Figure 5 is a side view of another embodiment of the structure according to the present invention, in closed position around a tubular organ, the fibers being activated,

Figure 6 represents another embodiment of the structure according to the present invention,

Figure 7 is a side view of another embodiment of the artificial structure according to the present invention,

Figure 8 is a cross-sectional view of structure of Figure 7, in closed position around a tubular organ, the fibers being inactivated, and

Figure 9 is a cross-sectional view of structure of Figure 7, in closed position around a tubular organ, the fibers being activated.

### Detailed description

In the present description, the terms "tubular part of an organ" means a region of an organ in the living body having an overall cylindrical shape, for example a blood vessel, the urinary tract, the colon, the stomach or any other body part against which pressure has to be applied.

In the present description, the term "pulsatory" means that each fiber is activated and deactivated very frequently in order to close or open very frequently the region of the tubular part around which the fiber has been placed. Each fiber is preferably activated or deactivated several times a day, and most preferably several times an hour. For example each fiber can be activated during one minute and deactivated during three minutes.

In the present description, the term " contractile material" means in particular Electro Active Polymers (EAP), Electro Active Ceramics (EAC), Shape Memory Alloys (SMA). Any suitable material can be used for the fibers. A suitable SMA material for the contractible fibers is Nitinol™. In this case the fibers can be stretched by as much as 4% when below the transition temperature, and when heated, they contract, recovering thereby to their original, shorter length with a usable amount of force in the process. Temperature range varies according to the fiber' s physical characteristic and is usually comprised between 37°C to 70°C.

The fibers can have a spiral form in order to improve the length of the shortening.

Other particularly interesting materials are Biometal fibers (BMF) commercialized by Toki Corporation Inc., Japan. Those materials are able to reversibly contract upon a controlled heating caused by the supply of an electric current/voltage.

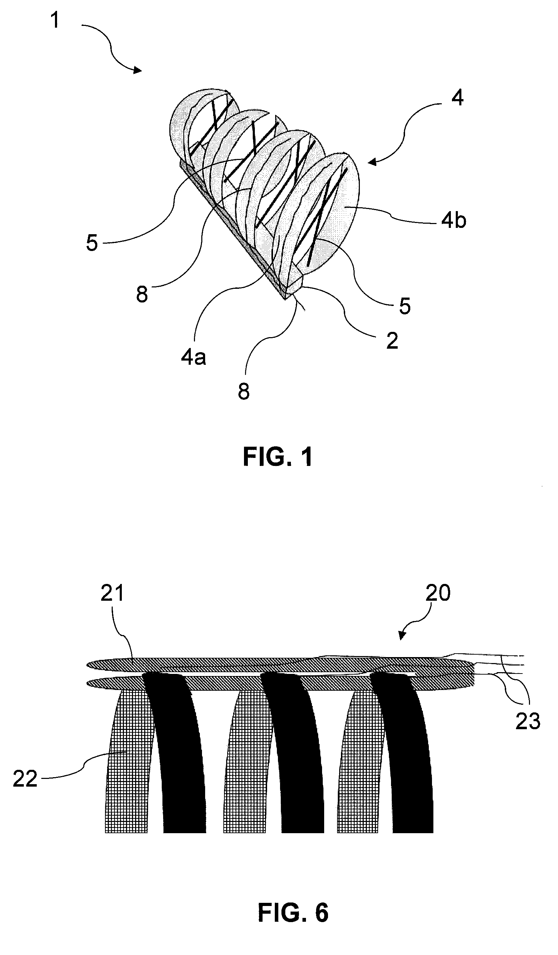

Referring to figures 1 to 4, one embodiment of an artificial contractile structure 1, used to treat urinary incontinence, comprises a rigid support 2 having the form of a clamp formed of two parts jointed by an hinge 3. The clamp is adapted to be placed around the tubular part of an organ to be contracted, for example the urethra. Each part comprises teeth 4, distributed along the support 2 to form four pairs of teeth 4a and 4b. For each pair of teeth 4a, 4b, a fiber 5 made of shape memory material is fixed to the inside of the teeth 4a, 4b in order to cross the area delimited by the associated teeth 4a and 4b. Therefore, each fiber 5 and its corresponding pair of teeth 4 allow to form four gates, each being able to constrict one region of the tubular part to close it, in a pulsating and alternating manner, and independently of each other.

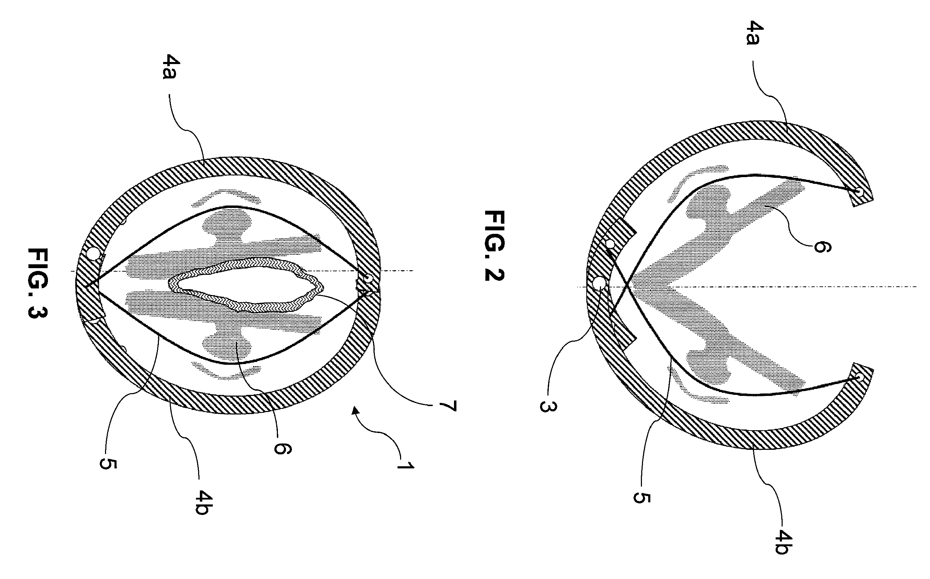

As shown by figure 2, each end of the fiber 5 is fixed to each tip of tooth 4a, 4b and the fiber is crossed at the bottom of the support 2. The fibers 5 are adapted to be non-rectilinear in the rest position, and to have a reduced length in the activated position. The length of the fiber is chosen so that the pressure of each fiber, when it is contracted, on the tubular part, is high enough to completely close the tubular part.

A protecting element 6 is disposed inside the clamp, above the fibers 5, in order to protect the tissue from the heating of the fibers 5 and to better distribute the pressure of the fibers to the tubular part of the organ. Such protecting element 6 can be made of silicone.

In the apparatus of the invention, the structure 1 is used with an activator comprising a microprocessor adapted to distribute current to fibers 5 by conducting wires 8 and to drive the contraction of the fibers in order to pulsatory and alternately contract said fibers. There are also means for opening on demand said artificial contractile structure, used by the patient to inactivate all the fibers of the structure and open all the gates of the structure, and an implantable source of energy, for example a rechargeable battery. A percutaneous energy transfer supply can be developed for battery recharge. The apparatus can further comprise sensing means selected from pressure, temperature and movement sensing means. Such sensing means can to be fixed to the support of the artificial contractile structure.

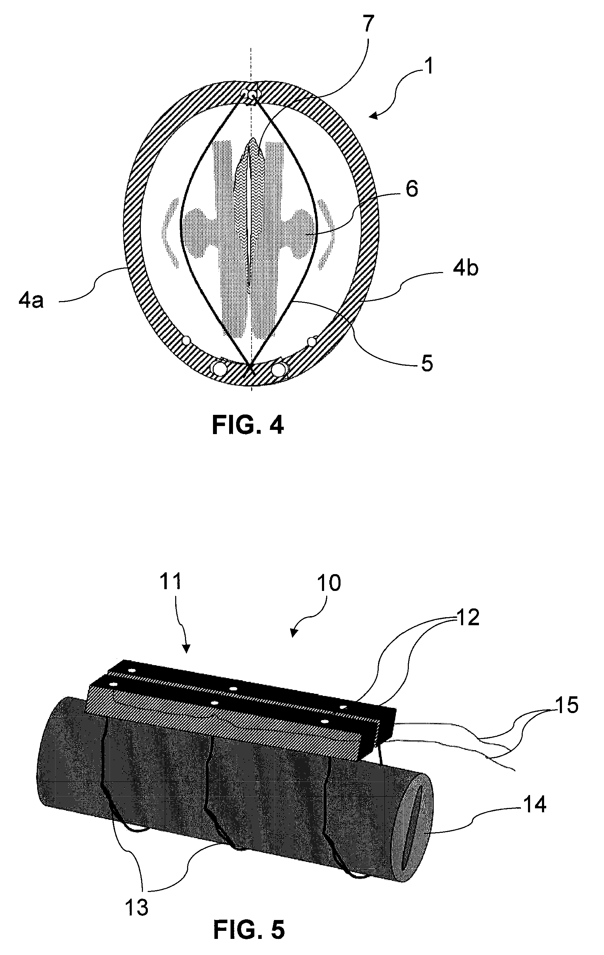

Referring to figures 3 and 4, the structure 1 is placed and fixed around an organ to close 7 and the clamp is closed. The fibers 5 are therefore distributed along the organ 7. In inactivated position, as shown by figure 3, one fiber 5 is in rest position and is not contracted. The region of the organ 7 around which the fiber 5 has been placed is not compressed and then not closed.

When an electric current/voltage is applied to the fiber 5 by the activator, the fiber 5 is activated and contracts, as shown by figure 4, in such a way as to reduce its length and then to reduce the diameter of the region of the organ 7 around which the fiber 5 has been placed, until to close said region of the organ 7. More precisely the fiber movement occurs in a plane which is transverse with respect to the direction of the tubular organ 7.

As there are at least two pairs of teeth, there are at least two gates formed by the teeth. The fiber of each gate is independently, pulsatory and alternately activated in order to contract one or the other region around which the fiber has been placed, in a pulsating and alternating manner. This allows an alternate contraction along said tubular part, several times an hour. Such a configuration avoids necrosis of the underlying tissue.

The activator is designed to activate at least one fiber so that at least one region of the tubular part is closed to avoid incontinence. The patient deactivates the apparatus if necessary, so that each fiber is inactivated to open each region of the tubular part of the organ, allowing the passage of the urine, for example.

Moreover, if the apparatus of the invention fails, there is no current in the fibers, which are therefore in the rest position. The structure 1 remains open. No surgery is necessary to allow the passage of the urine, for example.

Referring now to figure 5, another embodiment of the artificial contractile structure 10 comprises a support 11 comprising two parallel rods 12 to which three fibers 13 made of shape memory material are fixed by their ends. Each end of a fiber 13 is fixed to each rod 12 so that each fiber 13 forms a loop adapted to be placed around the tubular part 14 of an organ. The loops form three gates, each gate being able to constrict one of the three regions of the tubular part 14 to close it. As described above, each fiber 13 is linked to an activator by conducting wires 15, said activator driving the contraction of the fibers 13 in order to pulsatory and alternately contract them, so that each region of the tubular part is completely closed, in a pulsating and alternating manner, independently of each other.

Referring now to figure 6, another embodiment of the artificial contractile structure 20 comprises a rod 21 in U shape. Three strips 22 are fixed by their central part to the first leg of the U rod 21. Each leg of the U rod carries the current necessary to activate the strips. Preferably, such strips 22 are made of electroactive polymer, for example a ionic polymer-metal composite (IPMC). The second leg of the U rod 21 allows to clamp the strips 22 and the electric current to be applied to one side of the strips 22.

Each strip 22 forms a U adapted to be placed around the tubular part of an organ, the U fibers forming three gates, each gate being able to constrict one region of the tubular part to close it, in a pulsating and alternating manner. The strips 22 and the second leg of the U rod 21 are linked to an activator by conducting wires 23. As describes above, said activator is suitable to drive the contraction of the strips 22 in order to pulsatory and alternately contract them, so that each region of the tubular part is completely closed, in a pulsating and alternating manner, independently of each other.

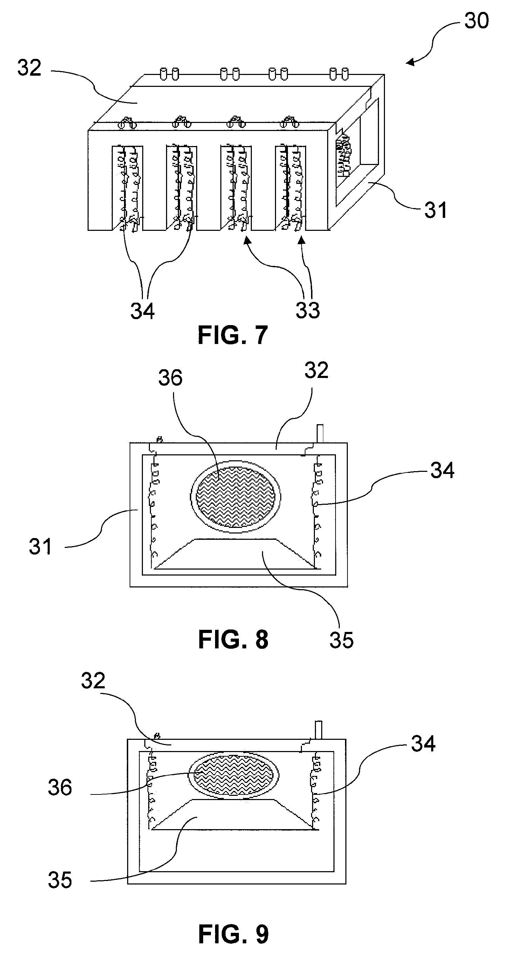

Referring now to figures 7 to 9, another embodiment of the artificial contractile structure 30 comprises a housing 31 adapted to be placed around the tubular part 36 of an organ. The housing 31 is closed by a sliding cover 32. The walls of the housing 31 comprise four openings 33 in which fibers 34 have been inserted. The fibers 34 are suspended by their ends to the upper wall of the housing 31. The central part of each fiber 34 comprises a press pad 35, placed opposite the sliding cover 32.

In inactivated position, as shown by figure 8, one fiber 34 is in rest position and is not contracted. The region of the organ 36 around which the fiber 34 has been placed is not compressed by the press pad 35 and then not closed.

When an electric current/voltage is applied to the fiber 34 by the activator, the fiber 34 is activated and contracts, as shown by figure 9, in such a way as to reduce its length and then to lift the press pad 35. The tubular part 36 is then compressed between the press pad 35 and the sliding cover 32 until to be completely closed.

As explained above, the fibers 34 and their press pads 35 form four gates, each gate being able to constrict one region of the tubular part 36 to close it, in a pulsating and alternating manner, in such a way as to avoid necrosis of the underlying tissue.

## Claims

1. Artificial contractile structure (1, 10, 20, 30) comprising a support (2, 11, 21, 31) and several fibers (5, 13, 22, 33) fixed to said support (2, 11, 21, 31), said fibers (5, 13, 22, 33) being made of a contractile material which can be activated by an activator in such a way as to provide said structure (1, 10, 20, 30) in a rest or in an activated position, the rest position being defined with non-rectilinear fibers and the activated position being defined with fibers of reduced length, said structure (1, 10, 20, 30) being adapted to be placed around a tubular part (7, 14, 36) of an organ to be contracted, wherein the fibers (5, 13, 22, 33) are distributed along said support (2, 11, 21, 31) in order to be able to reduce the diameter of said tubular part (7, 14, 36), when said fibers (5, 13, 22, 33) are contracted, in at least two distinct regions of the tubular part (7, 14, 36), and wherein the fibers (5, 13, 22, 33) are designed to be activated pulsatory and alternately, so as to completely close at least one region of the tubular part (7, 14, 36), in a pulsating and alternating manner.

2. Artificial contractile structure (1, 10, 30) according to claim 1, wherein the fibers (5, 13, 33) have at least two distinct parts fixed to the support (2, 11, 31).

3. Artificial contractile structure (1, 10) according to anyone of the previous claims, wherein the transition from the rest towards the activated position or vice-versa is defined by a fiber movement along a lateral direction which is perpendicular with respect to the fiber length.

4. Artificial contractile structure (1) according to anyone of the previous claims, wherein the support (2) is a hinged clamp adapted to be placed around the tubular part (7), comprising at least two pairs of teeth (4), and wherein a fiber (5) is fixed to the inside of each pair of teeth (4) in order to cross it so as to form at least two gates, each gate being able to constrict one region of the tubular part (7) to close it, in a pulsating and alternating manner.

5. Artificial contractile structure (10) according to anyone of claims 1 to 3, wherein the support (11 )comprises parallel rods (12) to which at least two fibers (13) are fixed by their ends, so that each fiber (13) forms a loop adapted to be placed around the tubular part (14) of the organ, the loops forming at least two gates, each gate being able to constrict one region of the tubular part (14) to close it, in a pulsating and alternating manner.

6. Artificial contractile structure (20) according to claim 1, wherein the support (21) comprises a U rod to which at least two fibers (22) are fixed by their central part, so that each fiber (22) forms a U adapted to be placed around the tubular part of the organ, the U fibers (22) forming at least two gates, each gate being able to constrict one region of the tubular part to close it, in a pulsating and alternating manner.

7. Artificial contractile structure (30) according to claim 1 or 2, wherein the support comprises a housing (31) adapted to be placed around the tubular part (36) of the organ, said housing (31) being closed by a sliding element (32), and wherein at least two fibers (33) are suspended by their ends to the support, the central part of each fiber (33) comprising a press pad (35) placed opposite the sliding element (32), the fibers (33) and their press pads (35) forming at least two gates, each gate being able to constrict one region of the tubular part (36) to close it, in a pulsating and alternating manner.

8. Use of the artificial contractile structure according to anyone of claims 1 to 7 for assisting or replacing a natural sphincter.

9. Apparatus comprising an artificial contractile structure as claimed in claims 1 to 7, wherein it further comprises an activator adapted to pulsatory and alternately contract the fibers so that said fibers completely close at least one region of the tubular part, in a pulsating and alternating manner, and means for opening on demand said artificial contractile structure.

10. Apparatus according to claim 9, wherein it further comprises sensing means selected from pressure, temperature and movement sensing means.

11. Apparatus according to the previous claim, wherein said sensing means are fixed to the support of the artificial contractile structure.

12. Apparatus according to anyone of the claims 9 to 11, wherein it further comprises an implantable source of energy.
